# EUROPÄISCHE PATENTSCHRIFT

(11) **EP 0 791 203 B1**
(45) Veröffentlichungstag und Bekanntmachung des Hinweises auf die Patenterteilung: **08.04.1998**
(21) Anmeldenummer: 95933328.7
(22) Anmeldetag: 06.10.1995
(51) Int. Cl.: G06F 19/00

(54) **INTEGRATIONSMEDIUM ZUM ERFASSEN, AUFBEREITEN, ÜBERTRAGEN UND ARCHIVIEREN VON ABRUFBAREN MEDIZINISCHEN DATEN EINES INDIVIDUUMS, DIE JEDERZEIT AN DIESEM VERFÜGBAR SIND**
INTEGRATION MEDIUM FOR CAPTURING, TREATING, TRANSMITTING AND STORING RECALLABLE MEDICAL DATA ON AN INDIVIDUAL WHICH ARE AVAILABLE ON THE LATTER AT ANY TIME
SUPPORT D'INTEGRATION POUR LA SAISIE, LE TRAITEMENT, LA TRANSMISSION ET L'ARCHIVAGE DE RENSEIGNEMENTS D'ORDRE MEDICAL ACCESSIBLES CONCERNANT UN INDIVIDU ET DISPONIBLES A TOUT MOMENT SUR LEDIT INDIVIDU

(30) Priorität: 10.11.1994 DE 4440178
(43) Veröffentlichungstag der Anmeldung: 27.08.1997
(73) Patentinhaber: CSB-System Software-Entwicklung & Unternehmensberatung GmbH, D-52511 Geilenkirchen (DE)
(72) Erfinder: SCHIMITZEK, Peter, D-52511 Geilenkirchen (DE)
(74) Vertreter: Haussingen, Peter
(86) Internationale Anmeldenummer: DE9501391
(87) Internationale Veröffentlichungsnummer: WO9615503

(56) Entgegenhaltungen:
- EP-A- 0 625 759
- WO-A-93/03449
- PROCEEDINGS OF THE INTERNATIONAL COMPUTER SOFTWARE AND APPLICATIONS CONFERENCE. (COMPSAC), CHICAGO, OCT. 31 - NOV. 2, 1990, Nr. CONF. 14, 31.Oktober 1990 KNAFL G, Seiten 82-87, XP 000223591 DIMITROFF D C ET AL 'AN OBJECT ORIENTED APPROACH TO AUTOMATING PATIENT MEDICAL RECORDS'
- PROCEEDINGS OF THE ANNUAL CONFERENCE OF THE ENGINEERING IN MEDICINE AND BIOLOGY SOCIETY, PHILADELPHIA, NOV. 1 - 4, 1990, Bd. 12, 1.November 1990 PEDERSEN P C;BANU ONARAL, Seiten 1262-1263, XP 000244755 MASAHIRO NISHIBORI ET AL 'AN AUTOMATED DATA CONVERSION AND ENTRY SYSTEM FOR THE MEDICAL OPTICAL CARD'

## Beschreibung

Die Erfindung betrifft ein Integrationssystem zum Erfassen, Aufbereiten, Übertragen und Archivieren von abrufbaren medizinischen Daten eines Individuums, die jederzeit an diesem verfügbar sind und es ist vorzugsweise einsetzbar zur digitalen Archivierung von Diagnosen und Befunden.

Nach der Offenlegungsschrift DE OS 42 13 797 ist ein medizinisches Informationssystem bekannt, das aus einem Datenträger und aus einem Datenauswertungsgerät besteht, indem der Datenträger in Format von Scheckkarten, die vom Patienten mitgeführt werden, ausgebildet ist. Die auf dem Datenträger vorhandenen Daten werden über Datenauswertungsgeräte ausgewertet bzw. modifiziert. Die Datenträger haben Risikoanzeigen, die auch ohne Datenauswertungsgeräte, erkennbar sind. Des weiteren enthält der Datenträger sichtbare Informationen, die eine Zuordnung des Datenträgers zum Patienten ermöglicht. Weiterhin ist der Datenträger mit z. B. einem Halbleiterspeicher versehen, der einen batteriegepufferten Schreib-Lesespeicher (RAM) oder einen elektrisch löschbaren und wiederbeschreibbaren Lesespeicher (RAM) besitzt. Die Steuerung der Eingabe/Ausgabe von Daten erfolgt u. a. durch einen Prozessor, der dem Datenträger zugeordnet ist.

Auf dem Datenträger sind Code abgespeichert, die zur Abfrage geschützter Daten über das Datenauswertungsgerät eingegeben werden müssen, ehe die Daten freigegeben werden. Das Datenauswertungsgerät besitzt eine Schnittstelle zur Verbindung mit einem externen Computer.

Der Nachteil dieser Lösung ist, daß die Daten auf dem Datenträger löschbar und somit nicht dokumentenecht sind. Dieses Löschen kann auch ungewollt durch äußere Einflüsse passieren. Eine intuitive Benutzerführung ist nicht gegeben, wobei das Auffinden von Daten durch den komplizierten Aufbau und durch die umständliche Bedienung des Datenauswertungsgerätes nicht leicht durchführbar ist.

Eine eindeutige Zuordnung von medizinischen Daten nach Facharztsektionen und die Wiedergewinnung dieser Daten aus den Facharztsektionen ist nicht gegeben.

Eine Archivierung von Befunden, die noch nicht in digitaler Form vorliegen und von herkömmlichen analogen bildgebenden Untersuchungsgeräten stammen, kann mit diesem Datenauswertungsgerät nicht vorgenommen werden bzw. von den Datenträger somit nicht erfaßt werden.

Aufgrund der geringen Speicherkapazität der hier angegebenen Lösung zur Realisierung des Scheckkarten-Datenträgers sind sowohl Standbilder als auch Bewegtbilder wegen ihres hohen Speicherbedarfs nicht archivierbar.

Mit der Offenlegungsschritt DE OS 35 34 638 ist eine Patientendatenkarte dargestellt, die aus einer Plastikhülle besteht, die in Personalausweisgröße ausgeführt ist und neben den Personendaten und Notfalldaten, die sofort lesbar angeordnet sind, einen Datenträger (z. B. eine Diskette) einsteckbar aufnimmt. Der Datenträger ist zur Sicherung der Identität des Patienten mit einem Laserstrahl codemäßig markiert. Die Patientendaten sind nach einem codierten System z. B. Anamnese, Allergien, EKG, Diagnose, Therapie, Laboruntersuchungen etc. auf dem Datenträger abgelegt.

Der Nachteil dieser Lösung ist bei den Anwendungsbeispielen Magnetbandspeicher und Mikroprozessor der gleiche, wie schon bei der Offenlegungsschrift DE OS 42 13 797 geschildert.

Beim Anwendungsbeispiel "Diskette" gelten auch alle Nachteile von der Offenlegungsschrift DE-OS 4213797, außer der zu geringen Speicherkapazität. Ein weiterer Nachteil bei der Lösung nach Offenlegungsschrift DE-OS 3534638 ist, daß der Datenträger nicht in Scheckkartenformat vorliegt.

In einer Literaturveröffentlichung "CANON EUROPA 1994" des Unternehmens Canon Europa N.V. Optikal Card Dept. Bovenkerkerweg 59-61 NL-1185 X B Amstelveen, Niederlande, ist eine Cardio Card dargestellt, auf der komplexe medizinische Informationen für jeden Patienten, einschließlich der Ergebnisse präoperativer Untersuchungen, der Operation selbst und von Folgeuntersuchungen aufgezeichnet werden. Die Karte besteht aus vier Teilen, von denen drei paßwortgeschützt sind. Diese Teile sind weiter unterteilt in vordefinierte Verzeichnisse je nach Art der Daten, wie Anamnese, Status, Befunde, Therapie, Angiogramme, SPECT-Bilder, Ultraschallbilder etc.

Es gibt zwei Versionen der Software, je nachdem, in welcher Umgebung die Karte benutzt wird. In den Hauptherzzentren die im allgemeinen ein hetrogenes PC/Mac-Netzwerk verwenden- wie die Karte als Speichermedium für das Krankenhausdatensystem Kor-Net benutzt. Auf der Karte wird IBM PC ASC II für alphanumerische sowie TIFF und JPEG für Bilddaten benutzt. Dadurch kann jeder Benutzer seinen Text- oder sein Bildverarbeitungsprogramm zum Schreiben und Lesen von Daten verwenden: Word Perfect, Photoshop etc. Kleinanwendungen zum Karteninitialisieren und -prüfen, Sammeln und Verteilen von Informationen im Netzwerk, zum Auswerten der Karte und um andere Kor-Net-Anwendungen über Aktivitäten hinsichtlich der Karte mitzuteilen sind inbegriffen. In kleineren Herzzentren kommt dann die Einzel PC-Version zum Einsatz. Auch hier kann die komplette Datenverwaltung verwendet werden, genauso wie separate Tools. Für die Multimediabearbeitung, wie bei MPEG-Daten, werden Elemente Dritter benutzt. Für die Diagnose- und Verfahrenskodierung werden ICD 9- und WHO-Kodierungen benutzt. Da die Kodierungen so umfangreich sind, wurden sie für die Kardiologie unterteilt.

Der Nachteil dieser Karte ist:
- daß Befunde, die im Ausland erstellt werden, nicht auf der Karte hinterlegt werden können,
- daß bei vererbbaren Krankheiten, die Vorfahren und Geschwister, die eine solche Karte besitzen, nicht auf in diesem Zusammenhang bestehenden Krankheiten überprüft werden können,
- daß die digitale Bildarchivierung für Arzte nicht möglich ist, die bis jetzt nur mit herkömmlichen analogen bildgebenden Untersuchungsgeräten gearbeitet haben,
- daß diese Karte keine Einteilung der medizinischen Daten nach Facharztsektionen besitzt, somit ist keine intuitive Benutzerführung vorhanden, die ein leichtes Auffinden dieser Daten ermöglicht,
- daß keine unterschiedlichen Datenformate in interne Formate umgewandelt und komprimiert auf der Karte hinterlegt werden und daß diese Daten auch nicht wieder in andere externe Systeme, in das jeweils gewünschte Format, exportiert werden.

Die Druckschrift WO 93/03449 beschreibt ein Archivesystem zum Speichern diskreter multimedialer medizinischer Daten auf einer Speicherkarte. Ebenfalls vorhanden ist ein Interface zum Empfangen der Signale bildgebender Geräte, ein Interface zum Erfassen einzelner Bilder aus dem bildgebenden Gerät, die Digitalisierung einzelner Bilder mittels Grabber -Karte, eine Zwischenspeicherung im RAM mit reversibler Komprimierung sowie die Ablage auf individuell selektierter diskreter Position auf der Speicherkarte. Alle Informationen sind später darstellbar und ausdruckbar. Die Bedienung erfolgt über eine graphische Benutzeroberfläche. Eine spezielle Bildkomprimierung (run length coding) und eine spezielle Bildkomprimierung (differentielle pulse code modelation) wird verwendet. Die Endspeicherung erfolgt über Laser auf einem nichtlöschbaren optischen Medium. Das Verfahren zur Speicherung diskreter med. Bilder (speziell Röntgen) auf der Speicherkarte und ein gleichzeitiges Drucken der gespeicherten Bilder sind beschrieben.

Der Nachteil dieser Lösung besteht darin, daß die Speicherkarte keine Notfalldaten beinhaltet, daß keine Verknüpfung zwischen Bild- und Tondaten vorgenommen wird, daß keine Teilung der medizinischen Daten nach Facharztsektionen vorliegt, daß die Daten nur unverschlüsselt vorliegen und daß die Karte nicht über einen Hochgeschwindigkeitsnetzanschluß mit beliebigen PIS/KIS-Systemarchiven verbunden werden kann.

Nach der Literaturveröffentlichung. Proceedings of the international computer software and applications conference (COMPSAC), Chicago, Oct 31-Nov.2, 1990, Mr. COMF. 14, Seiten 82-87 D.C. Dimitroff et al.: ,,An Object Oriented Approach To Automating Patient Medical Records" ist die Entwicklung eines tragbaren individuellen elektronischen automatischen Patientenberichtes, welches die Umwandlung der existierenden Papier-Berichte, der Berichte, die auf verschiedenen heterogenen Informationssystemen generiert wurden und die unterschiedlichen Ausgabemedien (Graphik, Bild, Ton) einiger moderner med. Geräte berücksichtigt.

Die Schrift offenbart einen medizinischer Patienten-Bericht. Als automatisierbarer med. Patientenbericht ist dieser objektorientiert in Form eines Multimedia-Datenbank-Systems auf einer Workstation programmiert. Die persönliche Datenbank ist auf WORM Speicher (als CD) abgelegt. Dadurch wird ein Mitführen der elektr. Datenbank, welche neben Diagnosen und Untersuchungen auch nichtmedizinische Daten (Kosten und soziales) enthält, allen primären Nutzern direkt elektronisch zugänglich. Über ein Netz ist sie indirekt allen sekundären Nutzern zugänglich. Die Workstation ist dazu mit einer optischer Leseeinrichtung versehen, wobei die Workstation zusätzlich in übergeordnete Netzwerke integriert ist. Vertrauliche Informationen sind nur für autorisierte Nutzer zugänglich. Patienten können eigene Daten zu Hause betrachten. Die Integration zu med. Gerätesystemen, zu zentralen med. Datenbanken ist aufgezeigt. Eine schnelle Bildrückgewinnung wird durch einen speziellen Algorithmus (2d-Strings) erreicht. Durch die Verwendung eines Gitters zur Klassifizierung wird eine heuristische Ordnung erreicht. Die zusätzlich zum internen Format mögliche Verwendung von coded und non-coded Information (z.B. Word-Text und Scanner) dient der Erfassung möglichst vieler Formate. Ebenso wird ein interface zwischen verschiedenen Speicherformen (z.B. analog oder digital) beschrieben. Bezüglich der Betriebssysteme werden viele Standards unterstützt, so daß viele Systeme sich die Daten teilen können. Dazu sind spezielle Treiber für ein und dieselbe CD entwikkelt wurden.

Der Nachteil dieser Lösung besteht darin, daß diese Lösung kein Speichermedium in Checkkartengröße besitzt, welches leicht mitführbar ist. Weiterhin ist keine triggergesteuerte Bildaufnahme möglich. Die Patentdaten werden nicht durch eine Verschlüsselung geschützt.

Die Aufgabe der Erfindung ist es, ein Integrationssystem zum Erfassen, Aufbereiten, Übertragen und Archivieren von abrufbaren medizinischen Daten eines Individuums, die jederzeit an diesem verfügbar sind, zu entwickeln, das die Nachteile des Standes der Technik beseitigt, und vorzugsweise die digitale Archivierung von Diagnosen und Befunden absichert, die nicht unbedingt in digitaler Form anliegen und deren wechselseitige Übertragung in andere Systeme ermöglicht, indem diese archivierten Diagnosen und Befunde nicht nur durch Bild und Textdaten ausgewiesen werden, sondern durch Tonsignale unterlegt sein sollen. Gleichzeitig muß eine intuitive Benutzerfährung möglich sein.. Die archivierten Bilder sind als Stand- und Bewegtbilder zu hinterlegen und als solche sollen sie abrufbar sein, wobei sie den relativen Gesundheitszustand und den veränderten Gesundheitszustand ausweisen müssen. Weiterhin sind die Stand- und Bewegtbilder durch Vor- und Rücklaufdarstellungen abrufbar im Archiv zu hinterlegen.

Erfindungsgemäß wird die Aufgabe durch die in dem Patentanspruch 1 angegebenen Merkmale gelöst. Bevorzugte Weiterbildungen der Erfindung ergeben sich aus den Unteransprüchen.

Die Vorteile der Erfindung bestehen darin,
- daß die digitale Archivierung von Diagnosen und Befunden ermöglicht wird, die nicht unbedingt in digitaler Form vorliegen müssen und deren wechselseitige Übertragung in andere externe Systeme möglich ist,
- daß die Einteilung der medizinischen Daten nach standardisierten Facharztsektionen vorgenommen wird und dadurch eine intuitive Benutzerführung vorhanden ist, und ein leichtes Ablegen und Auffinden der Daten möglich ist,
- daß gleichzeitig eine eindeutige Zuordnung der medizinischen Daten nach Facharztsektionen gegeben ist,
- daß unterschiedliche Datenformate in interne Formate umgewandelt und komprimiert auf der Archivkarte hinterlegbar sind, die auch wieder in andere externe Systeme und in das jeweils gewünschte Format exportiert werden können,
- daß durch die offene Architektur des Systems unterschiedliche Systeme miteinander kommunizieren können,
- daß Befunde, die im Ausland erstellt wurden, in das Kartenarchiv hinterlegt werden können,
- daß in Fällen von vererbbaren Krankheiten auch Vorfahren und Geschwister, die ein solches personengebundenes Archiv besitzen, auf in diesem Zusammenhang bestehende Krankheiten überprüfbar sind,
- daß die archivierten Daten, insbesondere die medizinischen Daten, die in Form von Stand- und/oder Bewegtbildern den relativen Gesundheitszustand der Organe des Individuums anzeigen (der Ist-Zustand der einzelnen Organe, der dem Individuum eigen bzw. angeboren ist und nicht der, der vom Arzt als Ideal-Zustand (hypothetischer Zustand) vorgegeben wird) und im Vergleich mit Daten der Untersuchung und Analyse zur Feststellung von relativen Veränderungen (Erkrankungen) benutzt werden,
- daß diese Daten im Archiv nicht löschbar hinterlegt sind,
- daß die Stand- und Bewegtbilder durch Vor- und Rücklaufdarstellung vom Archiv abrufbar zur Verfügung stehen, wobei die Bewegtbilder auch in Zeitlupe bis hin zum Einzelbild darstellbar sind,
- daß die Bild-, Ton- und Textdaten entsprechend des Protokolls für Hochgeschwindigkeitsnetze absetzbar bzw. abforderbar sind,
- daß alle Bild-, Ton- und Textdatenübertragungseinheiten integrierbar sind,
- daß eine triggergesteuerte Bildarchivierung vorhanden ist,
- daß die Archivierung schon mit dem Untersuchungsvorgang beginnt,
- daß eine grafiktähige Text- und Layoutverarbeitung möglich ist,
- daß die Daten ausdruckbar sind.

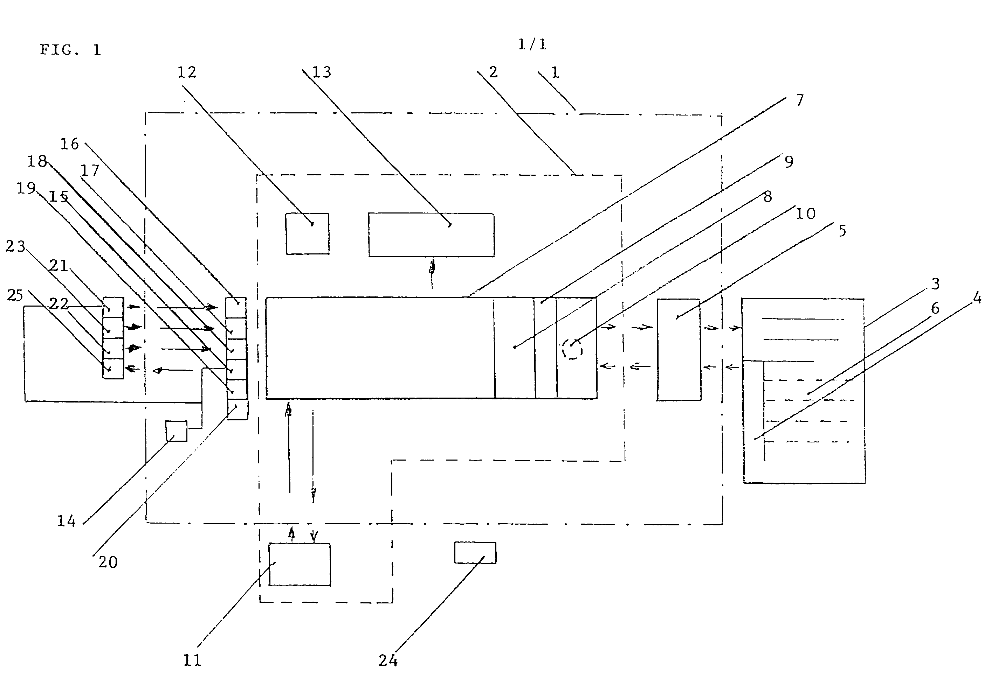

Das Integrationssystem wird anhand der Figur 1 erläutert.
Das Integrationssystem besteht aus einer Patientenkartensystem-aufbereitungseinheit 1, die ein Patienteninformations-/Krankenhausinformations-system (PIS-/KIS-system) 2 integriert und mit einer bekannten Karte 3, die in Scheckkartengröße ausgeführt ist und ein Archiv 4 besitzt, über eine bekannte Schreib- und Lesevorrichtung 5 in Wirkverbindung steht, indem
- die Karte 3 in bekannter Weise neben den persönlichen Daten des Individuums, die sichtbar sind, Notfalldaten, Risikofaktoren, die nicht geschützt sind und ein Archiv 4 für medizinische Daten, die geschützt sind, besitzt, wobei dem Archiv 4 Facharztsektionen 6 zugeordnet sind, die eine standardisierte Einteilung besitzen und das die hier hinterlegten medizinischen Daten unlöschbar sind,
- daß PIS-/KIS-System 2 aus einem Rechner 7 mit einem Arbeitsspeicher 8, aus einer Grabber-Karte 9 und aus einer eigens für die Patientenkartensystemaufbereitungseinheit 1 entwickelte Software 10 besteht, dem ein PIS-/KIS-System -Archiv 11, eine Tonwiedergabeeinrichtung 12 und ein Bildschirm 13 zugeordnet sind, wobei die Schreib- und Lesevorrichtung 5 nicht integrierter Bestandteil des PIS-/KIS-System ist,
- die Patientenkartensystemaufbereitungseinheit 1 die Schreib- und Lesevorrichtung 5 integriert und das PIS-/KIS-System-Archiv 11 ausschließt und daß sie eine Fußtaste 14, ein Triggersystem 15, eine Signalwandlereinheit 16, eine Tonwandlereinheit 17, eine Bildwandlereinheit 18, einen Hochgeschwindigkeitsnetzanschluß 19 und einen Drukkeranschluß 20 besitzt, indem ihr ein Signalgeber 21, der rückkoppelbar mit dem Triggersystem 15 ist und von dem medizinischen Signal z. B. EKG-Signal oder andere Signale übernommen werden können, ein bildgebendes Gerät 22, ein tongebendes Gerät 23, Hochgeschwindigkeitsnetze 24 und ein Drucker 25 zuordbar sind.

Die Funktion der Patientenkartensystemautbereitungseinheit 1 ist dadurch charakterisiert, daß im Archiv 4 der Karte 3 abrufbare medizinische Daten gesichert und unlöschbar hinterlegt werden können. Durch die standardisierte Einteilung der Facharztsektionen 6 ist eine eindeutige Zuordnung der abrufbaren medizinischen Daten zu den Facharztsektionen 6 gegeben und es ist eine intuitive Benutzerführung gewährleistet, wobei die medizinischen Daten vorzugsweise Stand- und Bewegtbilder vom ursprünglichen relativen Gesundheitszustand des Individuums und Befunde über dessen Veränderungen beinhalten, die vom Arzt von dem PIS-/KIS-System 2 erfaßt werden, die entweder dem Arbeitsspeicher 8 mit dem bildgebenden Gerät 22, direkt digital oder vom dazu gehörenden jeweiligen PIS-/KIS-System-Archiv 11 direkt digital in bekannter Weise über die Schreibund Leseeinheit 5 in die entsprechenden Facharztsektionen 6 des Archivs 4 übertragbar sind und bei nicht vorhandenem PIS-/KIS-System 2 vom bildgebenden Gerät 22 die analogen Daten, nach Umwandlung dieser in digitale Daten, in unterschiedlichen Datenformaten vorliegend, in interne Formate umgewandelt und komprimiert auf der Karte 3 im Archiv 4 hinterlegbar sind, die wiederum in andere externe Systeme, in das jeweilige gewünschte Format, exportiert werden können.

Bei einer Verknüpfung des Rechners 7 mit dem bildgebenden Gerät 22 werden alle Bilder, die digitalisiert im PIS-/KIS-System-Archiv 11 in unterschiedlichen Formaten anliegen, in interne Formate, den Facharztsektionen 6 zuordbar, umgewandelt.

Bei nicht vorhandener Verknüpfung des Rechners 7 mit dem bildgebenden Gerät 22 wird diese mit einer Grabber-Karte 9 hergestellt und die digitalisierten Bilder werden direkt in interne Formate, den Facharztsektionen 6 zuordbar, umgewandelt.

Durch den Einsatz der eigens entwickelten Software 10 im Rechner 7 wird die Schreibund Lesevorrichtung 5 mit dem Rechner 7 in der Weise integriert, daß die Karte 3 als ein fachübergreifendes Archiv nutzbar ist. Befunde, die im Ausland erstellt wurden, sind in das medizinische Archiv aufnehmbar. In Fällen von vererbbaren Krankheiten können Vorfahren und Geschwister, die ein solches medizinisches Archiv besitzen, auf die in diesem Zusammenhang bestehenden Krankheiten des Individuums überprüft werden.

Weiterhin sind die medizinischen Daten, insbesondere die Daten, die in den Facharztsektionen 6 archiviert sind, nicht löschbar.

Die medizinischen Daten, die als Stand- und/oder Bewegtbilder abrutbar archiviert sind, können durch Vor- und Rücklaufdarstellungen am Bildschirm 13 zur Verfügung gestellt werden, wobei die Bewegtbilder auch in Zeitlupe bis hin zur Einzelbildpräsentation darstellbar sind.

Nach Bedarf und Erfordernis können parallel zur Aufnahme von Stand- und Bewegtbildern als medizinische Daten Tondaten (BIO-Signale und/oder Kommentare des Fachmannes) durch das dafür bestimmte tongebende Gerät 23 bzw. Textdaten durch die Tastatur des Rechners 7 gemeinsam und zeitgleich mit den Bildern aufgenommen werden und in den Arbeitsspeicher 7 zwischengespeichert werden.

Anschließend wird über die Bedienung der Fußtaste 14 die Ablage der medizinischen Ton-, Bild- und/oder Textdaten in die entsprechenden Facharztsektionen 6 auf der Karte 3 vorgenommen, wobei diese auch parallel und zeitgleich mit der Aufnahme der Bilder auf der Karte 3 archiviert werden können. Durch die Bedienung der Fußtaste 14 sind diese medizinischen Daten wieder abrufbar und können auf dem Bildschirm 13 angezeigt werden, wobei die Tondaten an der Tonwiedergabeeinrichtung 12 anliegen.

Mit Hilfe des Hochgeschwindigkeitsnetzanschlusses 19 sind Voraussetzungen für die Patientenkartensystemaufbereitungseinheit 1 gegeben, die gespeicherten medizinischen Bild-, Ton- und/oder Textdaten entsprechend des Protokolls für Hochgeschwindigkeitsnetze extern abzusetzen bzw. extern abzufordern. Somit besteht die Möglichkeit auf der Karte 3 medizinische Daten eines Individuums, von einem externen PIS- oder KIS-System 2, die dort vorliegen, noch nachträglich dem Archiv 4 der Karte 3 zuzuordnen.

Weiterhin ist durch das Trigger-System 15 der Patientenkartensystemaufbereitungseinheit 1 die Möglichkeit gegeben, eine triggergesteuerte Bildspeicherung und somit dessen Archivierung auf der Karte 3 abrutbar abzusichern.

Des weiteren können, wenn notwendig, gleichzeitig oder zeitversetzt über den Drucker 25 ein oder mehrere Bilder oder Bilder mit Text nach Facharztsektionen ausgedruckt werden.

### Verwendete Bezugszeichen

- 1: Patientenkartensystemaufbereitungseinheit
- 2: PIS-/KIS-System
- 3: Karte
- 4: Archiv
- 5: Schreib- und Lesevorrichtung
- 6: Facharztsektionen
- 7: Rechner
- 8: Arbeitsspeicher
- 9: Grabber-Karte
- 10: Software
- 11: PIS-/KIS-System-Archiv
- 12: Tonwiedergabeeinrichtung
- 13: Bildschirm
- 14: Fußtaste
- 15: Triggersystem
- 16: Signalwandlereinheit
- 17: Tonwandlereinheit
- 18: Bildwandlereinheit
- 19: Hochgeschwindigkeitsnetzanschluß
- 20: Druckeranschluß
- 21: Signalgeber
- 22: bildgebendes Gerät
- 23: tongebendes Gerät
- 24: Hochgeschwindigkeitsnetz
- 25: Drucker

## Patentansprüche

1. Integrationssystem zum Erfassen, Aufbereiten, Übertragen und Archivieren von multimedialen medizinischen Daten eines Individuums, die jederzeit an diesem verfügbar sind, welches die wechselseitige Übertragung zwischen Formaten und Systemen unterstützt, bestehend aus folgenden Komponenten:
einem PIS-/KIS-System (2), welches ein PIS-/KIS-System-Archiv (11), einen Bildschirm (13), eine Tonwiedergabeeinrichtung (12) sowie einen Rechner (7) mit einem Arbeitsspeicher (8), einer Grabber-Karte (9) und einer Software (10), beinhaltet, einer Schreibund Lesevorrichtung (5), einer Karte (3) mit nichtgeschützten Notfalldaten und einem geschützten Archiv (4) für medizinische Daten, sowie aus geeigneten Mitteln zur triggergesteuerten Eingabe/Ausgabe multimedialer Daten in den Arbeitsspeicher (8) des PlS-/KlS-Systems (2), indem das PIS/KIS-System (2) über eine Signalwandlereinheit (16) mit einem Signalgeber (21), über eine Tonwandlereinheit (17) mit einem tongebenden Gerät (23), über eine Bildwandlereinheit (18) mit einem bildgebenden Gerät (22), über einen Druckeranschluß (20) mit einem Drucker (25) und über ein Triggersystem (15) sowohl mit einer Fußtaste (14) als auch rückkoppelbar mit dem Signalgeber (21) verbunden ist,
- wobei die Grabber-Karte (9) analoge Bilddaten in digitale Bilddaten umwandelt, welche anschließend in dem Bildspeicher (8) des Rechners (3) geladen werden können, indem dieser Vorgang nur in dem Fall vorgenommen wird, bei welchem die vom Untersuchungsgerät (22, 23) kommenden analogen Daten nicht bereits durch eine A/D-Wandlereinheit (16; 17; 18) digitalisiert wurden sind,
- wobei das PIS-/KIS-System (2) über Datenübertragungsschnittstellen mit externer EDV und über eine Schreib- und Lesevorrichtung (5) mit der Karte (3) in Wirkverbindung steht wodurch das PIS-/KIS-System (2) triggergesteuerte multimediale Daten zwischen dem Arbeitsspeicher (8) und dem Archiv (4) der Karte (3) austauschen kann, -wobei die multimedialen Daten über Verschlüsselungs- und Komprimierungsroutinen der Software (10) hybrid in lesbarer und verschlüsselter Form auf der Karte (3) gespeichert werden, indem medizinische Daten in einem geschützten und in Facharztsektionen (6) unterteilten Archiv (4) verwaltet werden,
- wobei die Software (10) mittels Treiber für verschiedene PIS-/KIS-Systeme und mittels Konvertierungsprogrammen zw. unterschiedlichen Formaten eine einheitliche intuitive Benutzeroberfläche zum Erfassen, Aufbereiten, Übertragen uiid Archivieren beliebiger multimedialer Daten ermöglicht,dadurch gekennzeichnet,
daß die Karte (3), welche zusätzlich ohne Hilfsmittel lesbare Notfalldaten beinhaltet, mit optischem Speicher ausgeführt ist, der gewährleistet, daß die ständig mitführbare med. Datenbank eine ausreichende Datenmenge aufnehmen kann,
daß eine Patientenkartensystemaufbereitungseinheit (1) die Karte (3) primär als persönliche medizinische Datenbank verwendet, indem sie das zum PIS-/KIS-System (2) zugeordnete PIS-/KIS-System-Archiv (11) primär ausschließt und statt dessen die Schreib-Lesevorrichtung (5) der Karte (3) integriert,
daß die Karte (3) über die Patientenkartensystemaufbereitungseinheit (1) multimedial über einen Hochgeschwindigkeitsnetzanschluß (19) und ein Hochgeschwindigkeitsnetz (24) mit beliebigen internen oder externen PIS-/KIS-System-Archiven (11) Daten austauschen kann.

2. Integrationssystem zum Erfassen, Aufbereiten, Übertragen und Archivieren von multimedialen medizinischen Daten eines Individuums, die jederzeit an diesem verfügbar sind, nach Anspruch 1, dadurch gekennzeichnet, daß das Archiv (4) optional zusätzliche Angaben (Kosten und Sonstiges) beinhaltet, auf welche Routinen zur Kostenberechnung med. Dienstleistungen zurückgreifen

3. Integrationssystem zum Erfassen, Aufbereiten, Übertragen und Archivieren von multimedialen medizinischen Daten eines Individuums, die jederzeit an diesem verfügbar sind, nach Anspruch 1, dadurch gekennzeichnet, daß die triggergesieuerten multimedialen Daten der Karte (3) über das Hochgeschwindigkeitsnetz (24) mit denen anderer Individuen auf anderen medizinischen Datenbanken verglichen werden können, indem durch die Speicherung der Daten in einem nach Facharztsektionen (6) unterteilten indizierten Archiv (4) eine Verwaltung verwandschaftlicher Kreuzbezugnahmen möglich ist.

## Claims

1. Integration system for the capture, processing, transfer and storage of an individual's multimedia medical data, available here at all times, which supports mutual transfer between formats and systems, consisting of the following components:
a PIS/KIS system (2), which contains a PIS/KIS system archive (11), a monitor (13), a sound reproduction unit (12) and a PC (7) with a working memory (8), a grabber card (9) and software (10), a writing/reading device (5), a card (3) with non-protected emergency data and a protected archive (4) for medical data, and appropriate means for trigger-controlled input/output of multimedia data into the working memory (8) of the PIS/KIS system (2), whereby the PIS/KIS system (2) is connected via a signal converter unit (16) to a signal generator (21), via an acoustic converter unit (17) to an acoustic device (23), via an image converter unit (18) to an imaging device (22), via a printer connection (20) to a printer (25) and via a trigger system (15) to both a pedal switch (14) and, in a manner which can be coupled back, to a signal generator (21),
- whereby the grabber card (9) converts analog image data to digital image data which can then be loaded into the image store (8) of the PC (3), this process only being carried out if the analog data from the examination device (22; 23) has not already been digitised by means of an A/D converter unit (16; 17; 18),
- whereby the PIS/KIS system (2) is in operative connection via data transfer interfaces with external EDP and via a writing/reading device (5) with a card (3), the PIS/KIS system (2) being capable of exchanging trigger-controlled multimedia data between the working memory (8) and the archive (4) on the card (3),
- whereby the multimedia data is stored on the card (3) in a mixture of readable and encrypted forms by means of encryption and compression routines of the software (10), medical data being administered in a protected archive (4) which is subdivided by area of medical specialisation (6),
- whereby by means of drivers for different PIS/KIS systems and conversion programs for the different formats, the software (10) provides a uniform intuitive user interface for the capture, processing, transfer and archiving of any multimedia data, characterised by the fact that the card (3), which also contains emergency details which are can be read without the use of any aid, is fitted with an optical memory which guarantees that the portable medical database can accept a satisfactory volume of data,
a patient card system processing unit (1) uses the card (3) primarily as a personal medical database, whereby it primarily excludes the PIS/KIS system archive (11) assigned to the PIS/KIS system (2), instead incorporating the writing/reading device (5) of the card (3),
the card (3) can exchange multimedia data via the patient card system processing unit (1) using the high-speed network connection (19) and a high-speed network (24) with any internal or external PIS/KIS system archives (11).

2. Integration system for the capture, processing, transfer and storage of an individual's multimedia medical data, available here at all times, in accordance with Claim 1, characterised by the fact that the archive (4) has the option of containing additional details (costs and miscellaneous) which can be accessed by routines for costing medical services.

3. Integration system for the capture, processing, transfer and storage of an individual's multimedia medical data, available here at all times, in accordance with Claim 1, characterised by the fact that the trigger-controlled multimedia data on the card (3) can be compared with data pertaining to other individuals in other medical databases via the high-speed network (24), whereby the storage of the data in an indexed archive (4) which is subdivided by area of medical specialisation (6) makes possible the administration of genetic cross references.

## Revendications

1. Système d'intégration destiné à saisir, préparer, transférer et archiver des données médicales multimédiatiques d'un individu qui sont disponibles n'importe quand dans celui-ci, système assistant le transfert réciproque entre des formats et des systèmes et se composant de la façon suivante :
d'un système d'information patients et hôpitaux (2) qui comprend des archives (11) du système d'information patients et hôpitaux, un écran (13), une installation de restitution de sons (12) ainsi qu'un ordinateur (7) muni d'une mémoire de travail (8), d'une carte d'acquisition (9) et d'un logiciel (10), se composant d'un dispositif de lecture-écriture (5), d'une carte (3) munie de données d'urgence non-protégées et d'archives protégées (4) pour données médicales, ainsi que se composant de moyens appropriés destinés à l'entrée/sortie commandées par déclenchement des données multimédiatiques dans la mémoire de travail (8) du système d'information patients et hôpitaux (2) en reliant le système d'information patients et hôpitaux (2) par l'intermédiaire d'une unité de conversion de signaux (16) à un transmetteur de signaux (21), par l'intermédiaire d'une unité de conversion de sons (17) à un appareil restituant des sons (23), par l'intermédiaire d'une unité de conversion d'images (18) à un appareil restituant des images (22), par l'intermédiaire d'un branchement pour imprimante (20) à une imprimante (25) et par l'intermédiaire d'un système de déclenchement (15) aussi bien à une pédale (14) que de façon susceptible à être réinjecté à un transmetteur de signaux (21),
- la carte d'acquisition (9) conversant des données analogues d'images en données numériques d'images qui peuvent être chargées à la fin dans la mémoire d'images (8) de l'ordinateur (3) en exécutant cette procédure seulement dans le cas où les données analogues venant de l'appareil d'analyse (22;23) n'ont pas déjà été numérisées par une unité de conversion analogique/numérique (16;17;18),
- le système d'information patients et hôpitaux (2) étant relié de façon effective par l'intermédiaire de jonctions de transfert de données à une informatique externe et par l'intermédiaire d'un dispositif de lecture-écriture (5) à la carte, ce par quoi le système d'information patients et hôpitaux (2) peut échanger des données multimédiatiques commandées par déclenchement entre la mémoire de travail (8) et les archives (4) de la carte (3),
- les données multimédiatiques étant mémorisées sur la carte (3), de façon hybride sous une forme codée et susceptible d'être lue, par l'intermédiaire de routines de compression et de codage du logiciel (10) en gérant des données médicales dans des archives (4) protégées et divisées en sections de médecin spécialisé (6),
- le logiciel (10) permettant une interface d'utilisation uniforme et intuitive au moyen de programmes de gestions pour différents systèmes d'information patients et hôpitaux et au moyen de programmes de conversion entre différents formats afin de saisir, de préparer, de transférer et d'archiver des données multimédiatiques quelconques, **caractérisé par le fait**
que la carte (3), comprenant des données d'urgence susceptibles d'être lues sans adjonction de moyens de secours, est exécutée avec une mémoire optique qui garantit que la banque médicale de données susceptible d'être constamment entraînée peut recueillir une quantité suffisante de données,
qu'une unité de préparation du fichier patients (1) utilise la carte (3) au premier degré en banque de données médicale et personnelle en excluant au premier degré les archives (11) du système d'information patients et hôpitaux associé au système d'information patients et hôpitaux (2) et en intégrant au lieu de cela le dispositif de lecture-écriture (5) de la carte (3),
que la carte (3) peut échanger des données par l'intermédiaire de l'unité de préparation du fichier patients (1) et de façon multimédiatique par l'intermédiaire d'un branchement pour réseau à grande vitesse (19) et d'un réseau à grande vitesse (24) avec des archives (11) du système d'information patients et hôpitaux, au choix, externes ou internes.

2. Système d'intégration destiné à saisir, préparer, transférer et archiver des données médicales multimédiatiques d'un individu qui sont disponibles n'importe quand dans celui-ci, selon la revendication 1, **caractérisé par**
le fait que les archives (4) comprennent en option des indications supplémentaires (frais et autres) auxquelles des routines ont recours pour le calcul des frais de prestations de services médicaux.

3. Système d'intégration destiné à saisir, préparer, transférer et archiver des données médicales multimédiatiques d'un individu qui sont disponibles n'importe quand dans celui-ci, selon la revendication 1, **caractérisé par**
le fait que les données multimédiatiques commandées par déclenchement de la carte (3) peuvent être comparées par l'intermédiaire du réseau à grande vitesse (24) à celles d'autres individus sur d'autres banques médicales de données en permettant une gestion des références croisées apparentées en mémorisant les données dans des archives (4) indexées et divisées en sections de médecin spécialisé (6).
